# EUROPEAN PATENT SPECIFICATION

(11) **EP 2 995 318 B1**
(45) Date of publication and mention of the grant of the patent: **07.12.2022**
(21) Application number: 14184166.8
(22) Date of filing: 09.09.2014
(51) Int. Cl.: C08G 73/02, A61K 47/50

(54) **SYNTHESIS OF NANOPARTICULAR GENE CARRIER SYSTEMS BASED ON POLY(BETA-AMINOESTERS)**
SYNTHESE VON NANOPARTIKULÄREN GENTRÄGERSYSTEMEN AUF BASIS VON POLY(BETA-AMINOESTERN)
SYNTHÈSE DE SYSTÈMES NANOPARTICULAIRES PORTEURS DE GÈNES À BASE DE POLY (BÊTA-AMINOESTERS)

(43) Date of publication of application: 16.03.2016
(73) Proprietor: Istanbul Universitesi Rektorlugu, 34452 Istanbul (TR); Gok, Mehmet Koray, 34320 Istanbul (TR); Pabuccuoglu, Saadet, 34320 Istanbul (TR); Cevher, Erdal, 34116 Istanbul (TR); Demir, Kamber, 34320 Istanbul (TR); Pabuccuoglu, Serhat, 34320 Istanbul (TR)
(72) Inventor: Gok, Mehmet Koray, 34320 Istanbul (TR); Pabuccuoglu, Saadet, 34320 Istanbul (TR); Cevher, Erdal, 34116 Istanbul (TR); Demir, Kamber, 34320 Istanbul (TR); Pabuccuoglu, Serhat, 34320 Istanbul (TR)
(74) Representative: Mutlu, Aydin

(56) References cited:
- EP-A1- 0 002 457
- US-A1- 2004 071 654
- TANG SHAN ET AL: "Co-delivery of doxorubicin and RNA using pH-sensitive poly ([beta]-amino ester) nanoparticles for reversal of multidrug resistance of breast ca", BIOMATERIALS, vol. 35, no. 23, 3 May 2014 (2014-05-03), pages 6047-6059, XP028664975, ISSN: 0142-9612, DOI: 10.1016/J.BIOMATERIALS.2014.04.025

## Description

### Technical Field of the Invention

The present invention relates to the synthesis and characterization of novel biocompatible Poly(β-aminoester) compounds and nanoparticles (nPBAE) containing the same for use as gene carrier systems, which constitute an important application especially in the biotechnology related field of nanotechnology.

### Background of the Invention

The main purpose of the gene therapy is to overcome the genetic abnormality via transferring the genetic material to the targeted site of the patient. The pre-clinical or clinical gene therapy researches have been improved rapidly in order to provide the regeneration of the defective biological functions or maintenance of homeostasis for the last fifteen years.

Nowadays, gene therapy provides great hope especially for the treatment of genetic diseases, cancer, cardiovascular diseases and viral infection. Gene therapy aims not only to treat the diseases but also aims to transport of recombinant genetic materials to the nucleus allowing the gene expression wherein activated or deactivated protein synthesis occur. Transfection (gene transfer) is the transfer of a gene to the nucleus of another cell via various methods, such as electroporation, gene microinjection, viral gene transfer, non-viral gene transfer and the introduction thereof into the cell's DNA.

The carrier systems, which deliver gene for the gene expression, are called "vector". The vectors are divided into two main groups as viral and non-viral. The viral vectors carry out effective transfection due to their structures but the use of these vectors is limited because of their weak characteristics, such as cytotoxicity, viral combination etc. Because of their said negative characteristics, the researchers have tended to use non-viral vectors.

The researchers have developed new, targeted and non-viral gene carrier systems to be widely used in many applications such as transgenesis, process of introducing an exogenous gene into a living organism and gene therapy. However, some problems have been encountered such as the difficulties in the targeting of the gene carrier system, enzymatic degradation of the particles, cytotoxicity of chemical agents etc. Currently, the studies focus on solving these problems.

Nowadays, the non-viral vectors are used in a rate less than 25 percent and majority of use in clinical treatments is directed to the viral vectors. Since 1989 when the first gene therapy was performed, this approach is applied to more than 3000 patients by means of 600 procedures. (AMIJI, M.M., 2005, Polymeric Gene Delivery, CRC Press, London, 0-8493-1934-X.; KWON, G.S., 2005, Polymeric Drug Delivery, Taylor & Francis Group, Boca Raton, 978-0-8247-2532-7). The non-viral vector systems are not effective enough for the gene transfer, therefore there is no commercial products of these systems. These systems are divided into two groups as lipophilic and polymeric vectors. The cationic liposomes which are used before and during clinical studies, have the same toxicity values as viral vectors (TAIRA, K., KATAOKA, K., NIIDOME, T., 2005, Non-Viral Gene Therapy, Springer, 4-431-25122-7; WONG, S.Y., PELET, J.M., PUTNAM, D., 2007, Polymer Systems For Gene Delivery-Past, Present And Future, Progress in Polymer Science, 32, 799-837).

Various polymeric vectors, which are proved to be as efficient as viral vectors and some cationic carrier systems which are capable of forming ion complexes with a gene in anionic structure, have been developed in prior art (LUO, D.; SALTZMAN, W.M., 2000, Synthetic DNA delivery systems, Nat. Biotechnol. 18 (1), 33-37, VORHIES, J.S., NEMUNAITIS, J.J., 2009, Synthetic vs. Natural/Biodegradable Polymers For Delivery Of Shrna-Based Cancer Therapies. Methods in Molecular Biology, Macromolecular Drug Delivery, 480, 11-29). The common target of these studies is the synthesis of the polymeric carrier systems, which are as effective as viral vectors and have low toxicity. The most important advantage of polymeric vectors and polypeptides is the enhancement of their efficacy by allowing various modifications which improve the genetic material release characteristics of the polymers to the targeted site (THOMAS, M., KLIBANOV, A.M., 2003, Non-Viral Gene Therapy: Polycation-Mediated DNA Delivery, Applied Microbiology And Biotechnology, 62, 27-34). The other advantages of the cationic polymers are low toxicity, immune response sensitivity, and ease in production and stability (Mandke, R.; Singh, J. 2012, Cationic nanomicelles for delivery of plasmids encoding interleukin-4 and interleukin-10 for prevention of autoimmune diabetes in mice. Pharm. Res. 29, 883-897; Wu, G.C.; Zhou, F.; Ge, L.F.; Liu, X.M.; Kong, F.S. 2012, Novel Mannan-PEG-PE modified bioadhesive PLGA nanoparticles for targeted gene delivery. J. Nanomater. 2012, 1-9).

The transgenic polymers are preferred generally in gene delivery due to their ability to be easily modified. The transgenic polymers are divided into two groups; synthetic transgenic polymers and the natural transgenic polymers. The natural polymers generally used in gene delivery are the poly(aminoacids), such as poly-L-lysine (PLL), polyornithine, polyarginine etc., chitosan, dextran, collagen, gelatin and their modified derivatives (TIERA, M.J.; WINNIK, F.M.; 2006, FERNANDES, J.C., 2006, Synthetic and natural polycations for gene therapy: State of the art and new perspectives. Curr. Gene Ther. 6, 59-71; HOSSEINKHANI, H.; AZZAM, T.; TABATA, Y.; DOMB, A., 2004, Dextran-spermine polycation: an efficient nonviral vector for in vitro and in vivo gene transfection. Gene Ther. 11, 194-203; CHEVALLAY, B.; HERBAGE, D., 2000, Collagen-based biomaterials as 3D scaffold for cell cultures: Applications for tissue engineering and gene therapy. Med. Biol. Eng. Computing 38, 211-218; KAUL, G.; AMIJI, M. 2005, Tumor-targeted gene delivery using poly(ethylene glycol)-modified gelatin nanoparticles: in vitro and in vivo studies. Pharm. Res., 22, 951-961; KOMMAREDDY, S.; AMIJI, M., 2007, Antiangiogenic gene therapy with systemically administered sFlt-1 plasmid DNA in engineered gelatin-based nanovectors. Cancer Gene Ther. 14, 488-498). Similarly, according to Amiji, (AMIJI, M.M., 2005, Polymeric Gene Delivery, CRC Press, London, 0-8493-1934-X) the synthetic transgenic polymers can be classified as non-biodegradable synthetic transgenic polymers (polyethylene imine (PEI), polyethylene glycol (PEG) conjugates etc.), biodegradable transgenic synthetic polymers (poly(β-aminoesters (PBAE), polyamido amines, poly-imidazole etc.), polyethylene oxide/polypropylene oxide copolymers and polymeric polyethylene oxide, polyalkyl cyanoacrylate nano-microspheres.

PBAE, which is one of the prominent non-viral gene carriers among non-viral gene carrier systems, is obtained via Michael addition reaction between primer amine or bis-secondary amine groups and diacrylate groups. This method allows the presence of the biodegradable ester bonds and tertiary amine groups in the structure of synthesized polymer (SHEN, Y., TANG, H., ZHAN, Y., KIRK, E.A.V., MURDOCH, W.J., 2009, Degradable Poly(β-aminoester) Nanoparticles for Cancer Cytoplasmic Drug Delivery, Nanomedicine: Nanotechnology, Biology and Medicine, 5, 192-201, ZUGATES, G.T., ANDERSON, D.G., LITTLE, S.R., LAWHORN, I.E.B., LANGER, R., 2006, Synthesis of Poly(β-aminoester)s with Thiol-Reactive Side Chains for DNA Delivery, J. Am. Chem. Soc., 128, 12726-12734, KIM, T., SEO, H.J., CHOI, J.S., YOON, J.K., BAEK, J., KIM, K., PARK, J.S., 2005, Synthesis of Biodegradable Cross-linked Poly(β-aminoester) for Gene Delivery and its Modification, Inducing Enhanced Transfection Efficiency and Stepwise Degradation, Bioconjugate Chem.,16, 1140-1148).

Although the poly(amidoamine) (PAMAM), having a similar structure to that of PBAE, is synthesized in the aqueous solvents (protic), like water, alcohol etc., PBAEs are obtained in the anhydrous solvents (aprotic) like tetrahydrofuran (THF) or dichloromethane (DCM). The hydrolysis of ester bonds is prevented by the use of anhydrous solvents. The molecular weights of polymers based on number/weight distribution thereof are varying betweeen 2.000 and 50.000 depending on the solvent or monomer ratio. The PBAEs obtained in DCM, have usually higher molecular weight than the PBAEs obtained in THF (JEONG, J.H., KIM, S.W., PARK, T.G., 2007, Molecular Design of Functional Polymers for Gene Therapy, Prog. Polym. Sci.,32, 1239-1274).

The main reasons for preferring of PBAEs usage in gene transfer can be summerized as follows;
- The amine groups can be protonated in physiological pH (pH=7,4)
- They can be prepared with various monomers and may not necessitate a special purification method.
- The third and one of the most important properties of PBAEs is that in vivo applications PBAEs do not display any significant toxicity whereas PEI, a good transfection agent, displays high toxicity.

### Brief Description of the Figures

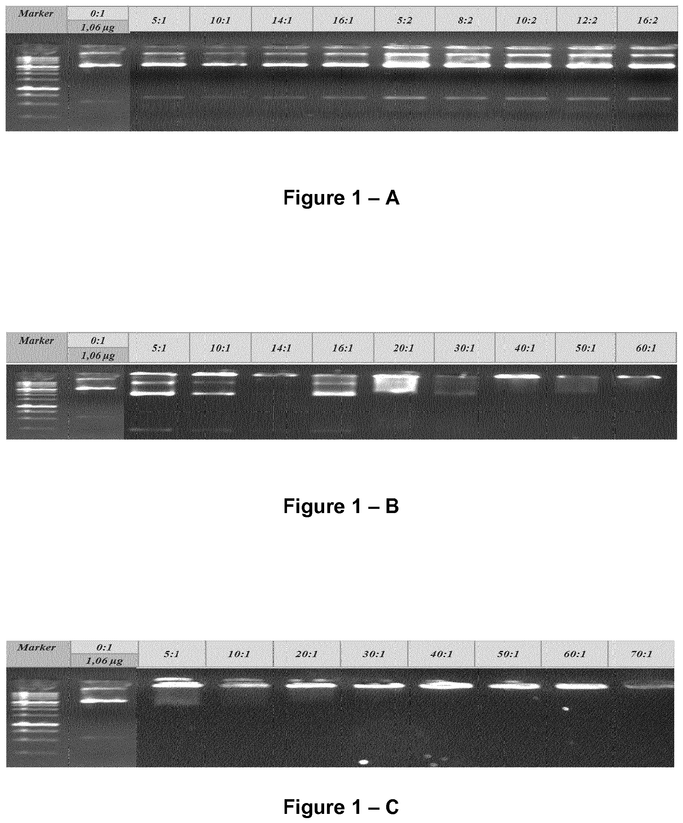

- **Figure 1** **- A:** Gel electrophoresis image of ignPBAE₁ formulation
- **Figure 1** **- B:** Gel electrophoresis image of ignPBAE₂ formulation
- **Figure 1** **- C:** Gel electrophoresis image of egnPBAE formulation

### Disclosure of the Invention

The present invention relates to the synthesis and characterization of novel biocompatible Poly(β-aminoester) compounds for use in gene carrier systems and to the development of suitable pharmaceutical forms preferably in the form of nanoparticles of poly(β-aminoester) compounds (nPBAE) for administering genes to a human and/or an animal.

In another embodiment, the present invention relates to the use of poly(β-aminoester) nanoparticles as gene carrier vectors in cell transfection, gene transfer and gene therapies.

Some terms and their abbrevations used throughout the description are given below:
PBAE : As used herein, the abbreviation "PBAE" refers to poly(β-aminoester).
nPBAE: As used herein, the abbreviation "nPBAE" refers to nanoparticles which are obtained from PBAEs.
ignPBAE: As used herein, the abbreviation "ignPBAE" refers to the complex formation of nanoparticles which is obtained from PBAE with the pDNA in different ratios (w/w).
egnPBAE: As used herein, the abbreviation "egnPBAE" refers to the complex formation of nanoparticles which is obtained from PBAE via the encapsulation method with pDNA in different amounts (w/w).

The inventors, in studies they have conducted, aimed to synthesize a poly(β-aminoester) compound and nanoparticles thereof not synhesized before, forming complexes with a plasmid DNA on desired levels, having approriate zeta potential value and having desired particle size.

At the end of the studies made, the inventors, have surprisingly seen that the synhesis of poly(β-aminoester) compound with desired characteristics is possible by the use of ethylenediamine as bis(secondary)amine and bisphenol-A-etoxylate diacrylate as bis(acrylate ester).

Accordingly, the poly(β-aminoester) compound of the present invention has the following formula: 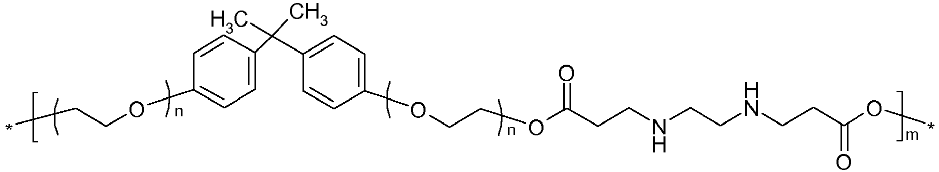
wherein n is an integer different than 0, and
m is an integer different than 0, and more preferably is inbetween 3 and 50,000.

The inventors have discovered that nanoparticles prepared with poly(β-aminoester) compound having the formula above form more stable complexes and higher efficiency is obtained by allowing higher gene expression after transfection.

In another aspect, there is provided a method for synthesis of poly(β-aminoester) compounds according to the present invention which mainly involves the reaction of ethylenediamine (EDA) and bisphenol-A-etoxylate diacrylate (BPAEDA) as shown in the following scheme: 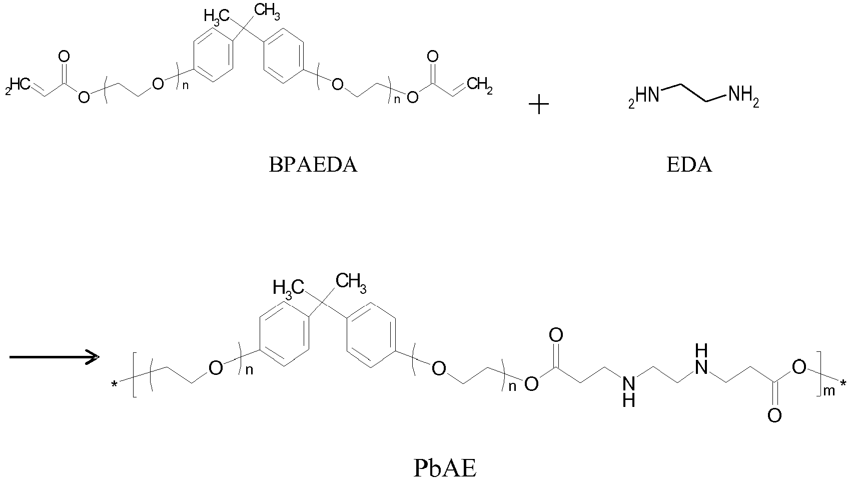
wherein n is an integer different than 0, and
m is an integer different than 0, and more preferably is inbetween 3 and 50,000.

In a preferred embodiment of this aspect, the method of the present invention comprises the steps of:
- dissolving bisphenol-A-etoxylate diacrylate and ethylenediamine monomers in a solvent and conducting the reaction inbetween the same,
- cooling the mixture and precipitating the same by a precipitation agent to obtain the PbAE compounds according to the present invention.

In a particular embodiment, the method according to this invention comprises the steps of:
- dissolving bisphenol-A-etoxylate diacrylate and ethylenediamine monomers separately in a solvent and adding ethylenediamine to the bisphenol-A-etoxylate diacrylate solution for reacting the same,
- cooling the mixture and precipitating the same by a precipitation agent, and
- drying the resulting product to obtain the PbAE compound according to the present invention.

The solvent used in the procedures above can be THF, DMSO or mixtures thereof with suitable amounts. The molar ratio of the reactants (BPAEDA:EDA) and solvent can be about 1:1, 1:1,1, and 1:2.

In another aspect, the present invention relates to nanoparticles prepared with poly(β-aminoester) compound of the above formula. These nanoparticles can be prepared by a method comprising dissolving of the compound according to the present invention in a solvent, and stirring the same until collidal particles are formed. It has been discovered that the nanoparticles prepared with the PbAE of the present invention are affected by the mixing rate applied during this process and that high stirring rate has a negative impact on particle size. The inventors noted unexpectedly that the stirring at a constant stirring rate below 8000 rpm, more preferably between 100-1000 rpm, and most preferably of about 650 rpm is beneficial for stability of the particles. The aforesaid solvent can be ethanol, DMSO or mixtures thereof, but excellent results were obtained with DMSO.

In another aspect of the present invention, provided is a complex comprising the nanoparticles mentioned above along with a pDNA (i.e. green fluorescense protein gene; GFP) for useful as a gene carrier vector in cell transfection, gene transfer and/or gene therapy. Such complexes can be prepared via ionic interaction or encapsulation methods as conventionally known. As shown in the examples in the context of the present invention, ionic interaction method carried out in DMSO as a solvent provides surprizing results, however the encapsulation method per se is found to be more effective.

The present invention is further elaborated within the context of the exemplary explanations given below with reference to the materials and methods used in synthesis of PbAE and nanoparticles thereof, which are tested according to certain aspects to show their useful effects. These exemplary explanations should nevertheless not be considered as limiting the scope of the appended claims.

### Examples

The monomers; bisphenol-A-ethoxylate diacrylate (BPAEDA) and ethylenediamine (EDA), which are used for the poly(β-aminoester) synthesis, were purchased from Sigma-Aldrich (USA). Tetrahydrofuran (THF) to be used as the solvent, and diethyl ether, as the precipitant, were obtained from Sigma (USA) and Merck (Germany), respectively. For the synthesis of the nanoparticle carrier system, ethanol (Merck; Germany), dimethylsulfoxide (DMSO) (Sigma; USA) and deionized water were used as solvents.

For the preparation of lysogen medium; yeast extract (Nitrogen Based, (-) free amino acids), Triptone (Bacto) and sodium chloride (for molecular biology) (Sigma) were used, whereas for the preparation of the competent bacteria cells; calcium chloride (CaCl₂) (for cell culture test) (Sigma) and glycerol (for molecular biology) (Sigma) were used.

Tris base (Sigma; USA), glacial acetic acid (Merck; Germany) and EDTA (Sigma; USA) for the preparation of Tris-Acetate-EDTA buffer (TAE, pH 8.0), agarose, ethidium bromide (for molecular biology) and bromophenol blue, which are purchased from Sigma; USA, were used for the gel electrophoresis experiments.

For the cell culture and gene transfection tests, Dulbecco modified eagle medium (DMEM) was used and for the preparation of this medium, L-glutamate, sodium pyruvate, sodium bicarbonate (NaHCO₃), penicillin and streptomycin (Sigma; USA) were used. Besides that, fetal calf serum (FCS) (Sigma; USA) was used for the FCS-containing DMEM (FCS+DMEM). For cell counting, trypan blue was used as the colorant (Sigma; USA).

### The Methods of PBAE Synthesis and Reaction System

Bisphenol-A-ethoxylate diacrylate (BPAEDA) and EDA monomers (5 mmol) were separately dissolved in THF (1:1, 1:1,1, 1:2 mole). Then EDA solution was added to the BPAEDA solution dropwise. The reaction was carried out in the inert atmosphere, for 48 hours at 50±1°C. Then the reaction solution was cooled to room temperature. It was purified via the addition of the PBAE onto anhydrous diethyl ether and elimination of the unreacted amine or acrylate monomers.. After that, PBAE is dried at room temperature under vacuum and store at 4 °C.

### The Characterization of PBAE

### Fourier Transform Infrared Spectroscopy (FTIR) Analysis

The structure of synthesized PBAE is identified by Fourier Transform Infrared Spectrophotometer (FTIR) technique. The analysis is performed using Digilab Excalibur-FTS 3000 MX model FT-IR apparatus. For FTIR analysis, tablets prepared by dilution where a sample/KBr ratio is of 1/200 are used and recorded at a wavenumber range of 400-4000 cm⁻¹.

### The Preparation of Nanoparticular Carrier Systems From PBAE

A series of pre-experiments are performed in order to obtain the desired and reproducible characteristics of nanoparticular systems.

The nanoparticular carrier systems are prepared via the nanoprecipitation or solvent displacement method (ethanol-water (nPBAE₁) or DMSO-water system (nPBAE₂)) from PBAE products. According to this method, PBAE polymer solution containing different rates of PBAE dissolved in DMSO or in ethanol is added dropwise to water at room temperature while stirring at a constant rate. Following to this step, the nanoparticle solutions are mixed for 1-5 hours. The experiments are performed with different stirring speeds for the examination of the effect of different stirring rates (650 rpm (nPBAE₃), 8000 rpm (nPBAE₄)) on the nPBAEs' particle characteristics. Moreover, the colloidal stability of the successful products are investigated for 4 weeks.

### The Preparation of Nanoparticles Carrying pDNA

According to the results of the analysis, nPBAEs, which have the most suitable properties, were treated with pDNA (i.e. green fluorescense protein gene; GFP) in different amounts (m/m) (ignPBAE). As a different method, nanoparticular systems are prepared via encapsulation method by treating PBAE products with pDNA in different ratios (w/w) (egnPBAE).

### Applied Tests and Instruments for nPBAE, ignPBAE and egnPBAE

### The Analysis of Zeta Potential, Particule Size and Polidispersity Index (PDI)

The zeta potential, particular size and PDI analyses of ignPBAE and egnPBAE are realized in deionized water at 25°C with Zetasizer Nano Series (Malvern Instruments, England) instrument. Therefore, the particles sizes of nanoparticles have been measured based on Dynamic Light Scattering (DLS) expressed in terms of Z-average. Polydispersity index of the nanoparticles is an indication of how narrow a Gaussian size distribution would be that could represent the fitted DLS data.

### Gel Electrophoresis Analysis of ignPBAE and egnPBAE

Produced complexes were analysed on gel electrophoresis in order to understand the complex formation ability between nanoparticles and pDNA (Cleaver Scientific Ltd., England). The agarose is dissolved by being boiled in Tris-acetate-EDTA (TAE, pH 8.0) solution until clear solution occured. Thereafter, it is cooled to the room temperature, and ethidium bromide (0.5 µg/mL) was added. Then the agarose gel was placed into the electrophoresis tank containing pH 8.0 TAE buffer. For analysis, sample:electrophoresis loading buffer in a ratio of 9:1 v/v was loaded on the agarose gel by mixing optionally with 6x agarose gel loading dye (bromphenol blue). The electrophoresis is completed in 1 hour under 100 V. After the electrophoresis, the agarose gels are evaluated by photos taken on UV transilluminator.

### Cytotoxicity Studies of nPBAE products and In Vitro Transfection Studies of ignPBAE, egnPBAE products

### Solutions

DMEM-Stock: For the solution preparation, the powder DMEM containing L-glutamate and sodium pyruvate is used. The stock solution was prepared with 13,38 g DMEM powder, 3,7 g NaHCO₃, 60 mg penicillin and 100 mg streptomycin.

FCS with DMEM (DMEM+FCS): DMEM+FCS medium (200 mL) was prepared with 174 mL DMEM Stock, 20 mL FCS, 2 mL Na-pyruvate, 2 mL L-glutamine and 2 mL non-essential aminoacid. DMEM+FCS medium is consumed within 1 week after preparation because of the lower stability of aminoacids and L-glutamine at +4°C.

FCS Without DMEM (DMEM-FCS): DMEM-FCS medium (200 mL) is prepared with 194 mL DMEM Stock, 2 mL Na-pyruvate, 2 mL L-glutamine and 2 mL non-essential aminoacid. DMEM-FCS medium is consumed within 1 week after preparation because of the lower stability of aminoacids and L-glutamine at +4°C.

### The Preparation of Primer Ovine Fibroblasts (POF), Primer Ovine Embryonic Fibroblasts (POEF), Human Embryonic Kidney (HEK293) Cells

### Thawing of Cells and Their Usage;

To be used in the studies, POF and POEF between 2 and 10 passages were obtained from sheep ear skin in the Istanbul University Faculty of Veterinary Medicine Department of Reproduction, and Artificial Insemination and HEK293 cells provided from Marmara University Faculty of Pharmacy Department of Basic Pharmaceutical Sciences were replicated and used.

Cryovials Cells in cryovials were removed from liquid nitrogen and were thawed in 37 °C water bath. To eliminate DMSO from the cells in the cryovial, the content is washed with 5 ml of DMEM and centrifuged at 2000 rpm for 5 minutes. Then the supernatant was discarded and the cells were dispersed within 1 ml of DMEM. The concentration of POF, POEF and HEK293 cells obtained concentration was measured by hemacytometric method with Thoma cell counting chamber. During counting by hemacytometric method, the viability of cells is also detected by using %0.4 trypan blue. After this procedure, the cells were plated on cell culture dishes at appropriate rate per unit cm² and were allowed to proliferate for 1 day.

The cells replicated and calculated with techniques described above to be used in the studies, were dispensed to petri dishes at appropriate concentration.

### Cytotoxicity Studies via Real Time Cell Analyser (RTCA)

The cytotoxicity studies of nPBAE were done with xCELLigence RTCA (Roche, Germany). The working principle of the apparatus is based on the measurement of electrical impedances of cells which were cultivated in gold plate (E-Plate 96) consisting 96 well, via gold electrodes. RTCA registers the electrical impedance changes which are caused by the cells numerical changes whereby the cell proliferation and viability are registered in real time. 100 µl cell culture medium was added into each of 96 well of E-Plate 96. E-Plate 96 is kept at room temperature for 30 minutes. At the end of the period, E-Plate 96 was placed into the xCELLigence and background value was measured.

Two different cell types: HEK293 and POF cells, were used in cytotoxicity studies. After measuring the background value, cells (30000) were plated into each well for 30 minutes at room temperature. At the end of the period, E-Plate is placed into the device and waited for one day for cell proliferation. After one day, the cells were controlled, then DMEM+FCS medium is removed and DMEM-FCS (200 µL) and nPBAE products in different concentrations were added into each wells. The products are allowed to contact with cells for 4 hours, and measurements were made with said device. At the end of 4 hours, DMEM-FCS and the products were removed, DMEM+FCS cell culture medium was added and cell proliferation was examined for 72 hours.

### The Transfection Efficiency Analysis of Nanoparticular Systems

According to all results of the analysis mentioned above, the transfection efficiencies of ignPBAE and egnPBAE products proved to be successful were assayed in HEK293, POF and POEF cells. 2000 cells thawed were plated onto each of 24 wells and incubated one day to proliferate at 37°C in CO₂ incubator. At the end of 1 day, cell proliferation was controlled with microscope and DMEM+FCS was removed. The nanoparticular systems and DMEM-FCS were added to the cells and the cells were incubated for 4 hours at 37°C in CO₂ incubator. After 4 hours, consumed DMEM-FCS was removed and then DMEM+FCS were added to the cells instead. The cells were incubated for 3 days at 37°C in CO₂ incubator. At the end of 3 days, the transfection by way of nanoparticle carriers, that is the transfection effeciency of the nanoparticle carriers was analyzed by way of Olympus IX71 reverse microscope under 460-480 nm fluorescence light that was emitted by the protein coded by pDNA. Based on the green fluorescence displayed by cells, transfected cells were determined and the numerical values were obtained by the examination of photographs of the specific regions on wells with the imaging system.

### Results and Discussion

### FTIR Characterization

The FTIR spectrum of the synthesized PBAE in ester structure, displays no big sharp band resulted from the stretching C=C bond present in vinyl groups of diacrylate compounds at approximately 1600-1680 cm⁻¹ region (max. at 1620 cm⁻¹). Moreover, the broad band at approximately 3100-3500 cm⁻¹ regions (max. at 3614 and 3370 cm⁻¹) is observed as a pique or shoulder in various intensities and maximums resulted from the stretching vibrations in the secondary amine group (CH2-NH-CH2) since the synthesized ester is in secondary structure. These results prove that the reactions between diacrylate compound and ethylenediamine have occurred and polymers were in ester structure as expected.

### The Optimization Results of Nanoparticles

Various experiments were carried out for investigation of the effect of using solvent in nanoparticle preparation and effect of high mixing speed on the properties of the nanoparticles. The nPBAE₁ nanoparticle with 38.9±5.33 mV zeta potential value, 108.9 nm particle size and 0.152 PDI, is convenient to be used in gene transfer applications as gene carrier. In order to examine the effect of solvent, DMSO was used instead of ethanol. To assure high mixing rate, the homogenizer is used at 8000 rpm. As seen in Table 1, high mixing speed caused the agglomeration of nanoparticles (nPBAE₃, nPBAE₄) and consequently, the particle size is increased. All these results show that nanoparticles prepared by using PBAE in DMSO (nPBAE₂), had the convenient features such as 179.4 nm particle size, 0.100 PDI value and high value of 54.5±8.64 mV zeta potential and that this formulation is covenient for transfection applications.

**Table 1: The Characteristics of Nanoparticle Formulations Based on Solvent Type and Mixing Rate**

| **Nanoparticle Formulation** | **Solvent Type** | **Mixing Rate (rpm)** | **Particle Size (nm)** | **Polydispersity** | **Zeta Potential (mV)** |
|---|---|---|---|---|---|
| nPBAE₁ | ETHANOL | 650 | 108.9 | 0.152 | 38.9±5.33 |
| nPBAE₂ | DMSO | 650 | 179.4 | 0.100 | 54.5±8.64 |
| nPBAE₃ | ETHANOL | 8000 | 155.7 | 0.222 | 30.7±4.51 |
| nPBAE₄ | DMSO | 8000 | 236.9 | 0.103 | 39.9±5.66 |

The colloidal stability of the nPBAE₁ and nPBAE₂ were investigated for 4 weeks (Table 2). An increase in the particle size and distribution of nPBAE₁ prepared with ethanol, especially starting from the second week and a decrease in the zeta potential value have been observed due to the instability of colloidal system. At week 4, besides the fact that the particle size of nPBAE₁ is increased to a very high value of 634.8 nm and a dispersity of 0.595 PDI, the zeta potential value thereof is decreased to -9.77±4.56 mV, thereby it is thought that these results were due to the degradation in the molecular structure of PBAE. Thus, it is concluded that the nPBAE₁ prepared according to this method have one-week shelf-life.

On the other hand, nPBAE₂ prepared with DMSO is stable for 4 weeks preserving almost the same values of particle size and dispersity. Although the zeta potential value of nPBAE₂ is decreased from 54.5±8.64 mV to 30.1±6.9 mV, this value is above 30 mV which signifies the colloidal stability of these systems. If this polymer is designed as a commercial transfection kit, it is possible that it can be marketed as a product having shelf life up to 1 month.

**Table 2: Change of The Characteristics of Nanoparticle Formulations in Time**

| **Nanoparticle Formulation** | **Period (week)** | **Particle Size (nm)** | **Polydispersity** | **Zeta Potential (mV)** |
|---|---|---|---|---|
| nPBAE₁ | 0 | 123.5 | 0.114 | 33.1±6.8 |
| nPBAE₁ | 1 | 136.0 | 0.167 | 29.7±4.39 |
| nPBAE₁ | 2 | 204.7 | 0.241 | 26.7±6.56 |
| nPBAE₁ | 3 | 353.7 | 0.367 | -0.92±7.90 |
| ηPBAE₁ | 4 | 634.8 | 0.595 | -9.77±4.56 |
| nPBAE₂ | 0 | 179.4 | 0.100 | 54.5±8.64 |
| nPBAE₂ | 1 | 179.5 | 0.073 | 52.5±7.72 |
| nPBAE₂ | 2 | 175.0 | 0.125 | 46.4±5.68 |
| nPBAE₂ | 3 | 169.7 | 0.070 | 36.7±5.71 |
| nPBAE₂ | 4 | 170.9 | 0.076 | 30.1±6.9 |

According to the results of studies made, it is understod that the nanoparticles prepared with the PBAEs of the present invention have the desired particle size, PDI and zeta potential values. The change of zeta potential values of the nanoparticles in time was examined and it has been observed that the zeta potential values of the nanoparticles prepared in DMSO stays at the desired level even after 4 weeks compared to the nanoparticles prepared in ethanol. Therefore, DMSO, provides both the desired characteristics of nanoparticles and the long-term preservation thereof.

It has been discovered that the nanoparticles prepared with the PBAEs of the present invention are affected by the mixing rate applied during this process and that high stirring rate has a negative impact on particle size.

### The Zeta Potential and Gel Electrophoresis Test Results of ignPBAE₁, ignPBAE₂ and egnPBAE

Zeta potential values of the ignPBAE products which are prepared by ionic interaction mechanism are given in Table 3, As seen in Table 3, only the formulation containing DNA displays negative zeta potential (-33,2 ± 7,49 mV), whereas the zeta potential value of ignPBAE₃₅ formulation is increased to positive values due to the ionic interaction between the nanoparticle and pDNA. Especially the zeta potential value of the formulation wherein the nanoparticle:pDNA ratio is 96,68:1, is above the threshold value of 30 mV, and this proves the colloidal stability of the system. On the other hand, it is observed that the zeta potential value is reached to positive values in the other ignPBAE₃₈ formulation wherein the nanoparticle:pDNA rate is 5,09:1. Accordingly, positive zeta potential values are obtained in the egnPBAE formulation wherein the nanoparticle:pDNA rate is 10:1.

When these results are evaluated together with the results of gel electrophoresis, it is observed that both results are consistent. It is understood that in the ignPBAE₁ formulation, only a part of the pDNA is complexed with nPBAE (Figure 1 - A). On the contrary, in ignPBAE₂ formulation having a polymer:DNA ratio of 20:1, it is observed that all of the pDNA is complexed with nPBAE (Figure 1 - B). In egnPBAE formulations, all pDNA is encapsulated wherein the PBAE:DNA ratio is 5:1 due to the encapsulation (Figure 1 - C).

**Table 3: The Zeta Potential Values and Nanoparticle:pDNA Ratios of Nanoparticle Formulations**

| **ignPBAE₁** | | **ignPBAE₂** | | **egnPBAE** | |
|---|---|---|---|---|---|
| **nPBAE:pDNA Ratio** | **Zeta Potential (mV)** | **nPBAE:pDNA Ratio** | **Zeta Potential (mV)** | **nPBAE:pDNA Ratio** | **Zeta Potential (mV)** |
| 0:1 | -33,2 ± 7,49 | 0:1 | -33,2 ± 7,49 | 0:1 | -47,7 ± 5,95 |
| 1,73 | -31,7 ± 6,99 | 0,85 | -16,4 ± 4,36 | 10 | 13,6 ± 5,05 |
| 3,46 | -29,3 ± 6,14 | 1,70 | -10,7 ± 6,06 | 30 | 19,1 ± 6,33 |
| 6,92 | -24,7 ± 5,24 | 3,40 | -7,8 ± 4,48 | 50 | 35,6 ± 7,14 |
| 10,38 | -21,7 ± 9,01 | 5,09 | 16,3 ± 5,40 | 60 | 40,9 ± 4,95 |
| 13,83 | -20,0 ± 10,8 | 6,79 | 28,2 ± 3,09 | | |
| 17,29 | -16,0± 9,13 | 8,49 | 29,9 ± 6,08 | | |
| 20,75 | -15,1 ± 10,00 | 10,19 | 29,1 ± 7,87 | | |
| 24,21 | -14,8 ± 7,74 | 11,89 | 29,7 ± 9,17 | | |
| 27,67 | -14,6 ± 3,44 | 13,58 | 36,2 ± 5,80 | | |
| 31,13 | -14,5 ± 4,63 | 15,28 | 36,5 ± 6,03 | | |
| 34,58 | -6,9 ± 7,98 | 16,98 | 39,4 ± 8,11 | | |
| 38,04 | -8,0 ± 4,48 | 18,68 | 45,3 ± 8,05 | | |
| 41,43 | -12,8 ± 7,02 | | | | |
| 44,89 | -12,7 ± 4,55 | | | | |
| 48,34 | -12,6 ± 3,44 | | | | |
| 51,79 | -10,1 ± 6,31 | | | | |
| 55,24 | -8,2 ± 5,66 | | | | |
| 58,70 | -7,9 ± 6,02 | | | | |
| 62,15 | -10,0 ± 4,65 | | | | |
| 65,60 | -12,3 ± 4,61 | | | | |
| 69,06 | -12,0 ± 3,55 | | | | |
| 72,51 | -4,4 ± 4,38 | | | | |
| 75,96 | -5,9 ± 4,06 | | | | |
| 79,42 | -9,9 ± 4,26 | | | | |
| 82,87 | -7,6 ± 4,71 | | | | |
| 85,90 | -4,5 ± 4,89 | | | | |
| 89,77 | -4,6 ± 4,13 | | | | |
| 93,23 | -0,7 ± 3,43 | | | | |
| 96,68 | 30,9 ± 10,60 | | | | |
| 100,13 | 40,4 ± 11,10 | | | | |
| 258,96 | 44,0± 7,15 | | | | |

Consequently, nanoparticles can be prepared with PBAE compounds according to the present invention and these can form complexes with pDNA via ionic interactions or encapsulation method.

Based on the results of the studies made by the inventors, complexes formed via ionic interactions and encapsulation method display different characteristics. Complexes formed via ionic interaction display different properties depending whether they are prepared in the presence of ethanol or DMSO. High amounts of PBAE are required to be used in complexes formed via ionic interaction in the presence of ethanol in order to obtain desired zeta potential values, in other words stability. However, complexes prepared via ionic interaction mechanism in the presence of DMSO have the desired zeta potential value, or stability, even at lower amounts of PBAE.

In this case, a nPBAE:pDNA ratio of more than 95, preferably between 95 and 260 would be required so that ignPBAE₁ complexes prepared via ionic interaction mechanism in the presence of ethanol have the desired stability. Accordingly, in a further embodiment of the invention, the nPBAE:pDNA ratio is more than 95, preferably between 95 and 260 in ignPBAE₁ nanoparticles prepared in the presence of ethanol via ionic interaction mechanism.

Moreover, the nPBAE:pDNA ratio can be between 4 and 30, preferably more than 5 and 25 so that ignPBAEₛ complexes prepared via ionic interaction mechanism in the presence of DMSO have the desired stability. Consequently, in another embodiment of the invention, the nPBAE:pDNA ratio can be between 4 and 30, preferably between 5 and 25 in ignPBAE₂ nanoparticles prepared in the presence of DMSO via ionic interaction mechanism.

egnPBAE nanoparticles prepared via encapsulation method do not require high polymer ratios for forming stable complexes with pDNA. The ratio of nPBAE:pDNA can be between 1 and 80, preferably 4 and 70, more preferably 5 and 60 so that these nanoparticles have the desired stability. In this aspect, in a further embodiment of the present invention, in egnPBAEs prepared with PBAEs according to the current invention via encapsulation method, nPBAE:pDNA ratio is between 1 and 80, preferably 4 and 70, more preferably 5 and 60.

### The Cytotoxicity Test of PBAE Based Nanoparticle Formulations

The cytotoxicity tests of PBAE were performed with two different types of cells HEK293 and POF) by RTCA method. The chitosan was used as a positive control group and PEI is used as a negative control group in these tests. For the investigation of the cytotoxic concentration of the polymers, both a polymer solution with the concentration level used in transfection studies (7,5 µg/mL) and with the concentration level which are highly above of that level were analysed in order to determine the most toxic level (25 µg/mL, 50 µg/mL, 150 µg/mL). The percentage of cell viability was calculated based on time-dependent cell index values are given by RTCA.

Based on the results of the cytotoxicity studies of PEI, the negative control group, performed with HEK293 cells, followed by the addition of samples on cells at 24^{th} hour, especially at a concentration level of 7,5 µg/mL, even at 28^{th} hour, it is observed that the viability decreases to a range of 40-50%. In general, in all concentration levels used at usual working conditions a decrease of 50% is observed. At the end of the 72^{nd} hours, although the cell viability is %90 and %60 at a PEI concentration of 7,5 µg/mL and 25 µg/mL respectively, higher toxicity is observed compared to the other modified synthesized polymers.

In case of PBAE polymer, an average decrease of 15% in cell viability is observed at all concentrations at the end of 28^{th} hour. However, at the end of 72^{nd} hour, especially at concentrations of 7,5 µg/mL and 25 µg/mL no toxic effect is observed in any HEK293 cells proving that said concentration levels are appropriate for transfection applications. The toxic effect is observed in higher concentrations (50 µg/mL and 150 µg/mL), high above than the concentrations used in transfection.

Based on the cytotoxicity results of PEI and PBAE products in POF cells, at 28^{th} and 72^{nd} hour the cell viability rates were found to be very low after the addition of PEI at 24th hour. However, PBAE polymer displays no toxicity at the concentration of 7,5 µg/mL, and a decrease in cell viability in each 28 hour is observed. Yet, depending on the concentration level of polymers, at the end of 72^{nd} hour, it is concluded that the toxicity values are compatible with transfection applications.

Based on the results of the studies made by the inventors, the concentration of the nPBAE solution to be used in transfection applications is between 5 µg/mL and 200 µg/mL, preferably 7 and 100 µg/mL, and more preferably 7,75 and 50 µg/mL. Accordingly, in another aspect, the concentration of the nPBAE solution to be used in transfection applications is between 5 µg/mL and 200 µg/mL, preferably 7 and 100 µg/mL, more preferably 7,75 and 50 µg/mL.

### The Transfection Efficiency Results of nPBAE Nanoparticular Systems

POF3 cell type having the highest transfection rate is used in the study and 25.000 cells are plated. The transfection efficieny was very low compared to other studies in literature despite the fact that the polymer:DNA ratio is 1,2:1 and a high amount of pDNA such as 10 µg is used. The main reason for this, as can be deduced from zeta potential value and gel electrophoresis findings, ignPBAE₁ formulation cannot form complexes with all pDNA used.

POF3 cell type having the highest transfection rate is examined for the transfection studies of ignPBAE₂ nanoparticles wherein the cell number is 25000. Although 1 µg gene is used, when the polymer:pDNA ratio is 68,1:1, the transfection efficiency is more efficient than ignPBAE₁ because as seen in the zeta potential and gel electrophoresis results of ignPBAE₂, all pDNA are complexed with all ignPBAE₂ for polymer:pDNA ratio which is 5,09:1.

The transfection studies of egnPBAE formulation were performed in HEK293 and POF cells wherein the transfection efficiency is the highest. In the case of where the cell number was 20.000, the polymer:pDNA ratio was 5:1 to 10:1, the amount of pDNA was 0,5 to 2 µg and the degree of transfection efficiency was 5. It is observed that this nanoparticle systems are more effective than those prepared by ionic interaction mechanism. As it can be deduced from the zeta potential value and gel electrophoresis results, the main reason for this is the complex formation ability of egnPBAE formulation with all pDNA used wherein the polymer:pDNA ratio is 10:1. Contrary to ignPBAE products adhesive through the adsorption on the surface, egnPBAE products encapsulate pDNA in the center of polyplex.

## Claims

1. A poly(β-aminoester) compound of the formula: 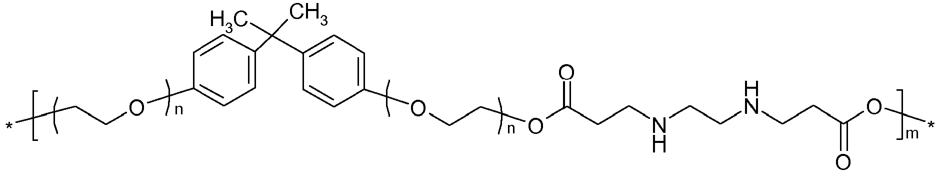 wherein m and n are integers different than 0.

2. A compound according to claim 1 which is in the form of nanoparticles.

3. A method for producing the compound of claim 1 comprising the steps of:
- dissolving bisphenol-A-etoxylate diacrylate and ethylenediamine monomers in a solvent and conducting the reaction inbetween the same, and
- precipitating the compound of claim 1 by a precipitation agent.

4. A method according to claim 3 wherein the solvent is selected from the group consisting of THF, DMSO and dichloromethane (DCM).

5. A method for producing the nanoparticles of claim 2 comprising the steps of:
- dissolving the compound of claim 1 in a solvent, and
- stirring this mixture until colloidal particles are formed.

6. A method according to claim 5 wherein stirring is conducted at a stirring rate below 8000 rpm.

7. A method according to claim 6 wherein the stirring rate is between 100-1000 rpm.

8. A method according to claim 5 wherein the solvent is selected from the group consisting of ethanol and DMSO.

9. A method according to claim 8 wherein the solvent is DMSO.

10. Nanoparticles of claim 2 for use as a gene carrier vector in cell transfection and gene transfer for gene therapy.

11. A complex comprising a nanoparticle according to claim 2 and a pDNA.

12. A method for producing the complex according to claim 11, comprising formation of said complex with a pDNA via ionic interaction or encapsulation method.

13. A method according to claim 12, wherein said method comprises formation of said complex via encapsulation method.

14. A method according to claim 12 wherein said method comprises formation of said complex via ionic interaction in the presence of DMSO.

15. A complex according to claim 11 for use in gene therapy of fibroblast cells in a human or animal.

## Patentansprüche

1. Poly(β-aminoester)-Verbindung der Formel: 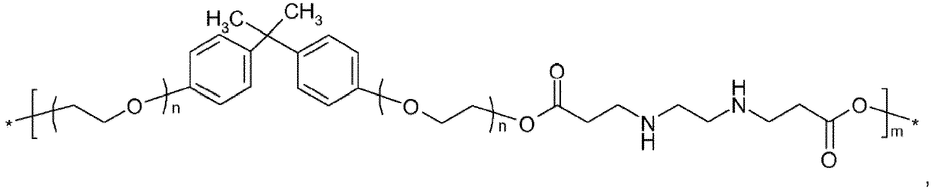 wobei **m** und **n** ganze Zahlen sind, die sich von 0 unterscheiden.

2. Verbindung gemäß Anspruch 1, die in Form von Nanopartikeln vorliegt.

3. Verfahren zur Herstellung der Verbindung gemäß Anspruch 1, das die folgenden Schritte umfasst:
- Lösen von Bisphenol-A-ethoxylat-diacrylat und Ethylendiamin-Monomeren in einem Lösemittel und Durchführen der Reaktion zwischen diesen, und
- Ausfällen der Verbindung gemäß Anspruch 1 mittels eines Fällungsmittels.

4. Verfahren gemäß Anspruch 3, wobei das Lösemittel ausgewählt ist aus der Gruppe bestehend aus THF, DMSO und Dichlormethan (DCM).

5. Verfahren zur Herstellung der Nanopartikel gemäß Anspruch 2, umfassend die Schritte:
- Lösen der Verbindung gemäß Anspruch 1 in einem Lösemittel, und
- Rühren dieses Gemischs, bis kollidale Teilchen gebildet sind.

6. Verfahren gemäß Anspruch 5, wobei das Rühren bei einer Rührgeschwindigkeit unterhalb von 8000 U/min durchgeführt wird.

7. Verfahren gemäß Anspruch 6, wobei die Rührgeschwindigkeit zwischen 100 und 1000 U/min beträgt.

8. Verfahren gemäß Anspruch 5, wobei das Lösemittel aus der Gruppe ausgewählt ist, die aus Ethanol und DMSO besteht.

9. Verfahren gemäß Anspruch 8, wobei das Lösemittel DMSO ist.

10. Nanopartikel gemäß Anspruch 2 zur Verwendung als ein Gen-Trägervektor bei einer Zelltransfektion und einem Gentransfer für die Gentherapie.

11. Komplex, der ein Nanopartikel gemäß Anspruch 2 und eine pDNA umfasst.

12. Verfahren zur Herstellung des Komplexes gemäß Anspruch 11, wobei das Verfahren die Bildung des Komplexes mit einer pDNA durch ionische Wechselwirkung oder ein Einkapselungsverfahren umfasst.

13. Verfahren gemäß Anspruch 12, wobei das Verfahren die Bildung des Komplexes durch ein Einkapselungsverfahren umfasst.

14. Verfahren gemäß Anspruch 12, wobei das Verfahren die Bildung des Komplexes durch ionische Wechselwirkung in Gegenwart von DMSO umfasst.

15. Komplex gemäß Anspruch 11 zur Verwendung in der Gentherapie von Fibroblastenzellen in einem Menschen oder in einem Tier.

## Revendications

1. Composé de poly(β-aminoester) de formule : 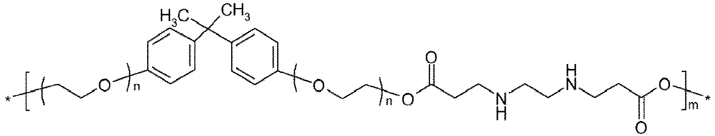 dans lequel m et n sont des nombres entiers différents de 0.

2. Composé selon la revendication 1 qui se trouve sous la forme de nanoparticules.

3. Procédé de production du composé selon la revendication 1 comprenant les étapes suivantes :
- dissolution de monomères de diacrylate de bisphénol-A-éthoxylate et d'éthylènediamine dans un solvant et réalisation de la réaction entre ceux-ci, et
- précipitation du composé selon la revendication 1 par un agent de précipitation.

4. Procédé selon la revendication 3 dans lequel le solvant est sélectionné dans le groupe constitué par le THF, le DMSO et le dichlorométhane (DCM).

5. Procédé de production de nanoparticules selon la revendication 2 comprenant les étapes de :
- dissolution du composé selon la revendication 1 dans un solvant, et
- agitation du mélange jusqu'à la formation de particules colloïdales.

6. Procédé selon la revendication 5 dans lequel l'agitation est réalisée à une vitesse inférieure à 8000 tr/min.

7. Procédé selon la revendication 6 dans lequel la vitesse d'agitation est comprise entre 100 et 1000 tr/min.

8. Procédé selon la revendication 5 dans lequel le solvant est sélectionné dans le groupe constitué par l'éthanol et le DMSO.

9. Procédé selon la revendication 8 dans lequel le solvant est le DMSO.

10. Nanoparticules selon la revendication 2 pour leur utilisation en tant que vecteur porteur de gène dans la transfection cellulaire et le transfert de gènes pour la thérapie génique.

11. Complexe comprenant une nanoparticule selon la revendication 2 et un ADNp.

12. Procédé de production du complexe selon la revendication 11, comprenant la formation dudit complexe avec un ADNp par l'intermédiaire d'une interaction ionique ou d'un procédé d'encapsulation.

13. Procédé selon la revendication 12, dans lequel ledit procédé comprend la formation dudit complexe par l'intermédiaire d'un procédé d'encapsulation.

14. Procédé selon la revendication 12 dans lequel ledit procédé comprend la formation dudit complexe par l'intermédiaire de l'interaction ionique en présence de DMSO.

15. Complexe selon la revendication 11 pour son utilisation en thérapie génique ou dans des cellules fibroblastiques chez un être humain ou un animal.
